# EUROPEAN PATENT APPLICATION

(11) **EP 4 170 678 A1**
(43) Date of publication of application: **26.04.2023**
(21) Application number: 20946016.1
(22) Date of filing: 23.07.2020
(51) Int. Cl.: G16H 50/20, G16H 30/20, G16H 30/40, G16H 50/70, G16H 50/50, G06N 3/08, G06N 20/00

(54) **METHOD FOR ANNOTATING PATHOGENIC SITE OF DISEASE BY MEANS OF SEMI-SUPERVISED LEARNING, AND DIAGNOSIS SYSTEM FOR PERFORMING SAME**

(71) Applicant: Deep Bio Inc., Seoul 08394 (KR)
(72) Inventor: LEE, Sang Hun, Seoul 08394 (KR); KIM, Sun Woo, Seongnam-si, Gyeonggi-do 13599 (KR)
(74) Representative: Flügel Preissner Schober Seidel
(86) International application number: PCT/KR2020/009721
(87) International publication number: WO 2022/019356

(57) **Abstract**

Disclosed in the present invention are a method for annotating a pathogenic site of a disease by means of semi-supervised learning. According to an aspect of the present invention, provided is the method comprising the steps in which: the diagnosis system using a neural network generates a patch-level classification neural network, which predicts a classification result relating to whether or not a predetermined disease is present in a patch, and a pixel-level classification neural network which predicts a classification result relating to the disease for each pixel constituting the patch; the diagnosis system obtains a plurality of slide images for learning, wherein each of the plurality of slide images for learning is labeled with a corresponding slide-level diagnosis result; and the diagnosis system gradually learns the patch-level classification neural network and pixel-level classification neural network by means of the plurality of slide images for learning.

## Description

### Technical Field

The present disclosure relates to a method for performing annotation on a pathogenic site of a disease using semi-supervised learning which enables patch-level and pixel-level semi-supervised learning to be performed using training data labeled with diagnosis results at the slide-level, and relates to a method for automatically performing annotation on a pathogenic site of a disease using a neural network trained through semi-supervised learning, and a diagnosis system performing the same.

### Background Art

One of the major tasks performed by pathology or department of pathology is to read a patient's biometric image (e.g., a biological tissue slide of the patient) to perform a diagnosis to determine the condition or symptom for a specific disease. Such diagnosis relies on the experience and knowledge of experienced medical personnel over a long period of time. Recent trends have increasingly been to read slide images generated by digital imaging of biological tissue.

Meanwhile, in recent years, due to the development of machine learning, attempts have been made to automate tasks such as recognizing and classifying images by computer systems. In particular, an attempt has been made to automate diagnosis performed by skilled medical personnel using a neural network (e.g., a deep learning method using a convolution neural network (CNN)), which is a kind of machine learning, and a typical example is image-based disease diagnosis through deep learning using a neural network (e.g., CNN). In particular, diagnosis through deep learning using a neural network (e.g., CNN) does not simply automate the experience and knowledge of conventionally skilled medical personnel, and in terms of finding the characteristic elements through self-learning and deriving the desired answer, there are cases in which the characteristics of disease factors that skilled medical personnel did not know are found in the image.

In general, diagnosis of disease through neural networks using biometric images uses a piece of a slide, i.e., patch (also referred to as a tile) which is a biometric image. In other words, a medical person skilled in the patch image may annotate the state of a specific disease (e.g., whether cancer has been manifested) or the pathogenic site of the disease, and trains the neural network by using these plurality of annotated patch images as training data. In this case, a convolution neural network may be used as the neural network.

However, in general, a task of creating a site annotation for annotating a pathogenic site in a patch image takes a lot of time and money compared to other annotation tasks. Therefore, it takes a lot of time and money to collect training data for training a neural network that performs prediction on a pathogenic site.

On the other hand, diagnosis of the entire slide is a general process of traditional medical practice, and training data labeled with slide-level diagnosis results may be obtained relatively easily from hospitals and the like. Therefore, there is an urgent need for a neural network capable of performing patch-level diagnosis on slide data tagged with slide-level diagnosis results that may be obtained relatively easily, and further, a method for training a neural network capable of predicting the pathogenic site of a disease by performing pixel-level diagnosis.

### [Prior Art Document]

- Patent Document : Korean Patent Application Publication No. 10-2016-0034814 "Client device with neural network and system including the same"

### Disclosure of the Invention

### Technical Goals

The technical problem to be achieved by the present disclosure is to train a patch-level neural network capable of predicting diagnosis results in patch units and a pixel-level neural network capable of predicting diagnosis results in pixel units by performing semi-supervised learning using only slide-level diagnosis results, and through this, to provide a method and system enabling annotation pathogenic sites of a disease.

### Technical Solutions

In accordance with an aspect of the present disclosure, there is provided a method including generating, by a diagnosis system using a neural network, a patch-level classification neural network configured to receive a patch that is a segmented part of a predetermined slide which is a biometric image and predict a classification result regarding whether or not a predetermined disease exists in the patch, and a pixel-level classification neural network configured to receive the patch and predict a classification result for the disease for each pixel forming the patch, acquiring, by the diagnosis system using the neural network, a plurality of slide images for training, each of the plurality of slide images for training being labeled with a corresponding slide-level diagnosis result, and gradually training, by the diagnosis system using the neural network, the patch-level classification neural network and the pixel-level classification neural network using the plurality of slide images for training, wherein the gradually training of the patch-level classification neural network and the pixel-level classification neural network includes: (a) for each of the plurality of slides for training, generating training data corresponding to the slide for training, wherein the training data corresponding to the slide for training includes patch-level training data for training the patch-level classification neural network and pixel-level training data for training the pixel-level classification neural network; (b) training the patch-level classification neural network using the patch-level training data corresponding to each of the plurality of slides for training; (c) training the pixel-level classification neural network using the pixel-level training data corresponding to each of the plurality of slides for training; and (d) repeatedly performing the operations (a) to (c) at least once.

In an embodiment, the generating of the training data corresponding to the slide for training may include: acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes; determining a representative patch image corresponding to the slide for training among the plurality of patch images corresponding to the slide for training, based on a prediction result for each of the plurality of patch images corresponding to the slide for training; and labeling the representative patch image corresponding to the slide for training with the slide-level diagnosis result of the slide image for training, thereby generating the patch-level training data corresponding to the slide image for training.

In an embodiment, the generating of the training data corresponding to the slide for training may include: acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes; determining a representative patch image corresponding to the slide for training among the plurality of patch images corresponding to the slide for training, based on a prediction result for each of the plurality of patch images corresponding to the slide for training; generating a mask corresponding to the representative patch image through gradient-weighted class activation mapping for the classification neural network that output a prediction result for the representative patch image; and labeling the representative patch image corresponding to the slide for training with the mask corresponding to the representative patch image to generate the pixel-level training data corresponding to the slide for training.

In an embodiment, the method may further include, by inputting a predetermined patch image to be diagnosed into the pixel-level classification neural network for which training has been completed, acquiring a classification result for the disease for each pixel forming the patch image to be diagnosed; and annotating a pathogenic site of the disease in the patch image to be diagnosed based on the classification result for each pixel forming the patch image to be diagnosed.

In an embodiment, the disease may be prostate cancer.

In accordance with another aspect of the present disclosure, there is provided a computer program recorded on a non-transitory computer-readable medium for performing the method described above which is installed in a data processing device.

In accordance with another aspect of the present disclosure, there is provided a diagnosis system using a neural network, including a processor, and a memory configured to store a computer program, wherein the computer program, when executed by the processor, causes the diagnosis system using the neural network to perform the method described above.

In accordance with another aspect of the present disclosure, there is provided a diagnosis system using a neural network including a storage module configured to store a patch-level classification neural network for receiving a patch that is a segmented part of a predetermined slide which is a biometric image and predicting a classification result regarding whether or not a predetermined disease exists in the patch, and a pixel-level classification neural network for receiving the patch and predicting a classification result for the disease for each pixel forming the patch, an acquisition module configured to acquire a plurality of slide images for training, each of the plurality of slide images for training being labeled with a corresponding slide-level diagnosis result, and a training module configured to gradually train the patch-level classification neural network and the pixel-level classification neural network using the plurality of slide images for training, wherein, in order to gradually train the patch-level classification neural network and the pixel-level classification neural network, the training module repeatedly performs a training process two or more times which includes, for each of the plurality of slides for training, generating training data corresponding to the slide for training, wherein the training data corresponding to the slide for training includes patch-level training data for training the patch-level classification neural network and pixel-level training data for training the pixel-level classification neural network, training the patch-level classification neural network using the patch-level training data corresponding to each of the plurality of slides for training, and training the pixel-level classification neural network using the pixel-level training data corresponding to each of the plurality of slides for training.

In an embodiment, the generating of the training data corresponding to the slide for training may include acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes, determining a representative patch image corresponding to the slide for training among the plurality of patch images corresponding to the slide for training, based on a prediction result for each of the plurality of patch images corresponding to the slide for training, and labeling the representative patch image corresponding to the slide for training with the slide-level diagnosis result of the slide image for training, thereby generating the patch-level training data corresponding to the slide image for training.

In an embodiment, the generating of the training data corresponding to the slide for training may include acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes, determining a representative patch image corresponding to the slide for training among the plurality of patch images corresponding to the slide for training, based on a prediction result for each of the plurality of patch images corresponding to the slide for training, generating a mask corresponding to the representative patch image through gradient-weighted class activation mapping for the classification neural network that output a prediction result for the representative patch image, and labeling the representative patch image corresponding to the slide for training with the mask corresponding to the representative patch image to generate the pixel-level training data corresponding to the slide for training.

In an embodiment, the diagnosis system may further include an annotation module configured to, by inputting a predetermined patch image to be diagnosed into the pixel-level classification neural network for which training has been completed, acquire a classification result for the disease for each pixel forming the patch image to be diagnosed, and annotate a pathogenic site of the disease in the patch image to be diagnosed based on the classification result for each pixel forming the patch image to be diagnosed.

### Advantageous Effects

According to the technical idea of the present disclosure, there is an effect of training a patch-level neural network capable of predicting diagnosis results in patch units and a pixel-level neural network capable of predicting diagnosis results in pixel units by performing semi-supervised learning using only slide-level diagnosis results, and through this, providing a method and system enabling annotation pathogenic sites of a disease.

### Brief Description of Drawings

In order to more fully understand the drawings cited in the detailed description of the present disclosure, a brief description of each drawing is provided.
FIG. 1 is a diagram illustrating an operating environment of a method for training a neural network using semi-supervised learning and performing annotation on a pathogenic site of a disease using the same in accordance with the technical idea of the present disclosure.
FIG. 2 is a diagram illustrating a schematic configuration of a diagnosis system in accordance with the technical idea of the present disclosure.
FIG. 3 is a flowchart illustrating a process of gradually training a patch-level classification neural network and a pixel-level classification neural network using a plurality of slide images for training by a diagnosis system in accordance with the present disclosure.
FIG. 4 is a diagram illustrating a specific example of a process of generating training data corresponding to one slide for training by a diagnosis system in accordance with the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. The terms are used only for the purpose of distinguishing one component from another.

The terms used in the present application are used only to describe a particular embodiment and are not intended to limit the present disclosure. Singular expressions include plural expressions unless the context clearly means otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail centering on embodiments of the present disclosure. Like reference numerals in each figure indicate like members.

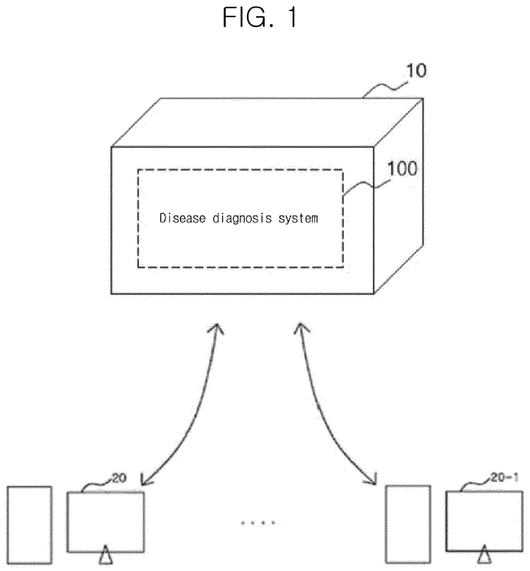

FIG. 1 is a diagram illustrating an operating environment of a method for training a neural network using semi-supervised learning and performing annotation on a pathogenic site of a disease using the same in accordance with the technical idea of the present disclosure. Referring to FIG. 1, the method according to the technical idea of the present disclosure may be performed by a diagnosis system 100.

The diagnosis system 100 according to the technical idea of the present disclosure may be a computing system. Alternatively, it may be installed in a predetermined server 10 to implement the technical idea of the present disclosure. The server 10 refers to a data processing device having an arithmetic capability for implementing the technical idea of the present disclosure, and in general, an average expert in the field of the present disclosure may easily infer that as well as data processing devices accessible to clients (terminals 20 to 20-1) via a network, any device capable of performing a specific service, such as a personal computer, a portable terminal, or the like, may be defined as a server.

The server 10 may include a processor and a storage device. The processor may refer to an arithmetic device capable of driving a program for implementing the technical idea of the present disclosure. The storage device may refer to a data storage means capable of storing programs and various data necessary for implementing the technical idea of the present disclosure, and may be implemented as a plurality of storage means according to embodiments. In addition, the storage device may include not only the main memory device included in the server 10, but also a temporary storage device or memory that may be included in the processor.

Although the diagnosis system 100 is shown as being implemented as any one physical device in FIG. 1, an average expert in the technical field of the present disclosure may easily infer that a plurality of physical devices may be organically combined as needed to implement the diagnosis system 100 according to the technical idea of the present disclosure.

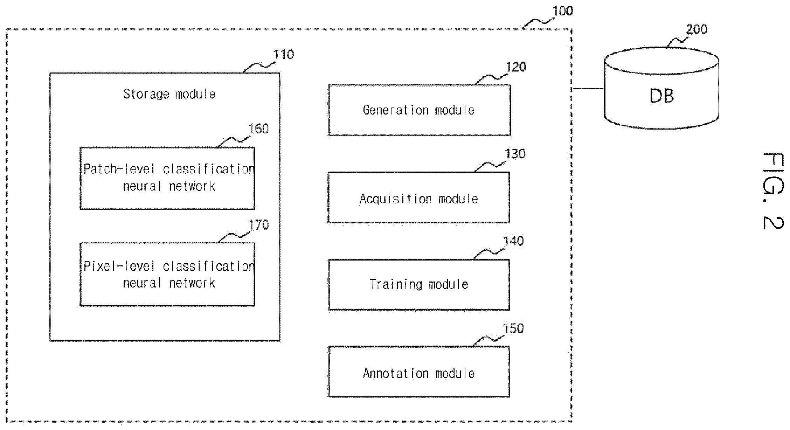

FIG. 2 is a diagram illustrating a schematic configuration of a diagnosis system 100 in accordance with the technical idea of the present disclosure.

Referring to FIG. 2, the system 100 may include a storage module 110, a generation module 120, an acquisition module 130, a training module 140, and an annotation module 150. According to embodiments of the present disclosure, some of the above-described components may not necessarily correspond to components essential to the implementation of the present disclosure, and the diagnosis system 100 may include more components than these according to embodiments. For example, the system 100 may further include a control module (not shown) for controlling functions and/or resources of other components (e.g. storage module 110, generation module 120, acquisition module 130, training module 140 and annotation module 150, etc.) of the system 100. In addition, according to embodiments, the system 100 may further include a database (DB) 200 for storing various information and/or data necessary to implement the technical idea of the present disclosure.

The system 100 may refer to a logical configuration having hardware resources and/or software required to implement the technical idea of the present disclosure, and does not necessarily mean a single physical component or a single device. In other words, the system 100 may refer to a logical combination of hardware and/or software provided to implement the technical idea of the present disclosure, and if necessary, it may be implemented as a set of logical components for implementing the technical idea of the present disclosure by being installed in devices spaced apart from each other and performing respective functions. In addition, the system 100 may refer to a set of components implemented separately for each function or role to implement the technical idea of the present disclosure. For example, the storage module 110, the acquisition module 120, the training module 130, and the annotation module 140 may be located in different physical devices or in the same physical device. In addition, according to embodiments, a combination of software and/or hardware constituting each of the storage module 110, acquisition module 120, training module 130, and annotation module 140 may also be located in a different physical device, and components located in different physical devices may be organically combined with each other to implement each of the modules.

In addition, in the present specification, a module may refer to a functional and structural combination of hardware for implementing the technical idea of the present disclosure and software for driving the hardware. For example, the module may refer to a logical unit of a predetermined code and a hardware resource for executing the predetermined code, and it may be easily inferred to an average expert in the field of the present disclosure that it does not necessarily mean physically connected code or one kind of hardware.

The DB 200 may store a plurality of slides for training that are pathological images. The slide for training may be various biometric images including tissue images and biopsy images. Each of the plurality of slides for training may be labeled with a slide-level disease diagnosis result.

For example, the disease may be prostate cancer, and hereinafter, prostate cancer will be mainly described, but those skilled in the art will easily understand that the technical idea of the present disclosure is not limited to prostate cancer.

Meanwhile, the slide-level diagnosis result labeled on each slide for training may be a judgment result of a disease determined by a medical professional in advance based on the corresponding slide.

For example, the diagnosis result of the slide may include, whether or not a specific disease has been manifested (negative/positive) as well as the degree of progression (or probability corresponding to the degree of progression) of the specific disease. For example, when the technical idea of the present disclosure is used for the diagnosis of prostate cancer, a Gleason pattern or Gleason score, which is an index indicating the degree of progression of prostate cancer, may be included in the diagnosis result. For example, the Gleason score has a value of 2 to 10, and a value of 6 to 10 may typically be considered cancer, and a larger number indicates a severe degree of prostate cancer expression. Gleason patterns can be classified into classes from 1 to 5.

The storage module 110 may store a patch-level classification neural network 160 and a pixel-level classification neural network 170, and the patch-level classification neural network 160 and the pixel-level classification neural network 170 may be generated by the generating module 120.

The patch-level classification neural network 160 may be a neural network for receiving a patch and predicting a classification result regarding whether or not a predetermined disease exists in the patch.

In this case, the patch may be a partial image obtained by dividing a certain slide into a certain size. In other words, one slide may be divided into a plurality of patches, and the patch-level classification neural network 160 may receive patches and perform patch-level diagnosis. Before performing the diagnosis, the patch-level classification neural network 160 may be previously trained by the training module 140.

Meanwhile, the information output by the patch-level classification neural network 160 performing patch-level diagnosis may be information enabling determination of whether a specific disease (e.g., a specific type of cancer) has been manifested in a tissue corresponding to the patch. For example, the information output by the patch-level classification neural network 160 may be information indicating a probability of whether a specific disease (e.g., a specific type of cancer) has been manifested in a tissue corresponding to the patch. In this case, if the probability value output by the patch-level classification neural network 160 is greater than or equal to a specific reference value (threshold value), it may be determined that the patch has a disease (e.g., prostate cancer).

According to an embodiment, the patch-level classification neural network 160 may output not only whether or not a specific disease has been manifested, but also information indicating the degree of progression of a specific disease (or the probability corresponding to the degree of progression). For example, when the technical idea of the present disclosure is used for diagnosis of prostate cancer, information output by the patch-level classification neural network 160 may include a Gleason pattern or a Gleason score, which is an index indicating the degree of progression of prostate cancer. Meanwhile, the threshold value used by the patch-level classification neural network 160 may be set in various ways, and according to the threshold value, a specific patch may be determined as a patch in which a disease has been manifested, i.e, a diseased patch, or a normal patch.

Meanwhile, the pixel-level classification neural network 170 may be a neural network for receiving a patch and predicting a classification result for the disease for each pixel forming the patch. The pixel-level classification neural network 170 that outputs the classification result for each pixel may perform segmentation to classify the pathogenic site of a disease.

The pixel-level classification neural network 170 may output whether or not a disease has onset or the probability of disease onset for each pixel forming a patch, and a site including a pixel determined to have an onset or a pixel having an onset probability exceeding a certain threshold value may be determined as a pathogenic site.

Depending on the embodiment, the pixel-level classification neural network 170 may predict whether or not a disease has onset (or the probability of disease onset) and/or the degree of progression of a disease (or probability corresponding to the above degree of progression) for each pixel and output the prediction.

Meanwhile, the pixel-level classification neural network 170 may be pre-trained by the training module 140 before performing segmentation (i.e., predicting diagnosis results for each pixel).

In this specification, a neural network may refer to a set of information representing a series of design matters defining a neural network. In an embodiment, the neural networks 160 and 170 may be convolutional neural networks.

As is well known, the convolutional neural network may include an input layer, a plurality of hidden layers, and an output layer. Each of the plurality of hidden layers may include a convolution layer and a pooling layer (or subsampling layer).

A convolution neural network may be defined by a function, filter, stride, weight factor, etc., for defining each of these layers. In addition, the output layer may be defined as a fully connected FeedForward layer.

The design details for each layer forming the convolution neural network are well known. For example, known functions may be used for each of the convolution function, pooling function, and activation function for defining the plurality of layers, the number of layers to be included in the plurality of layers, and separately defined functions may be used to implement the technical idea of the present disclosure.

An example of a convolution function is a discrete convolution sum. As an example of a pooling function, max pooling, average pooling, and the like may be used. An example of the activation function may be a sigmoid, a tangent hyperbolic (tanh), a rectified linear unit (ReLU) and the like.

When the design of the convolution neural network is defined, the convolution neural network in which the design is defined may be stored in the storage device. And when the convolution neural network is pre-trained, a weight factor corresponding to each layer may be specified.

In other words, the training of the convolution neural network may refer to a process in which the weight factors of each layer are determined. In addition, when the convolution neural network is trained, the trained convolution neural network may receive input data through an input layer and output output data through a predefined output layer.

The neural network according to an embodiment of the present disclosure may be defined by selecting any one or a plurality of widely known design items as described above, and a proprietary design may be defined for the neural network.

Referring again to FIG. 2, the acquisition module 130 may obtain training data for semi-supervised learning of the patch-level classification neural network 160 and the pixel-level classification neural network 170. In other words, the acquisition module 130 may acquire a plurality of slide images for training, and each of the plurality of slide images for training may be labeled with a slide-level diagnosis result corresponding thereto. For example, the acquisition module 130 may acquire a slide image for training from the DB 200.

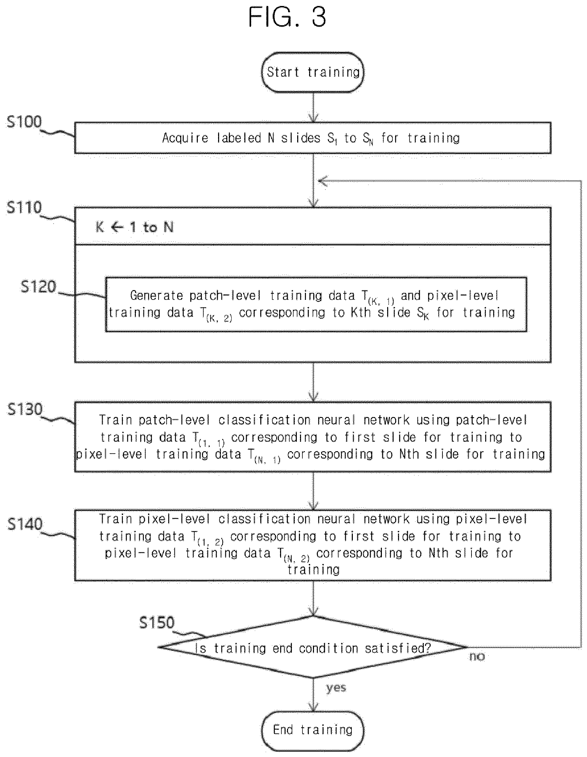

The training module 140 may gradually train the patch-level classification neural network 160 and the pixel-level classification neural network 170 using the plurality of slide images for training, and an example of an gradual training process is shown in FIG. 3.

Referring to FIG. 3, the acquisition module 130 may acquire N slide images S₁ to S_{N} labeled with slide-level diagnosis results (N is an integer greater than or equal to 2) (S100).

Thereafter, the training module 140 may perform a process of generating training data. More specifically, the training module 140 may generate training data corresponding to the slide fro training for each of the N slides for training (S110 and S120). At this time, each training data corresponding to the slide for training may include patch-level training data for training the patch-level classification neural network 160 and pixel-level training data for training the pixel-level classification neural network 170. For example, the training data corresponding to the Kth slide (where K is an integer where 1<=K<=N) may include patch-level training data T_{(K, 1)} and pixel-level training data T_{(K, 2)} (see S120).

When N data for training are generated through step S110, the training module 140 may train the patch-level classification neural network 160 by using N patch-level data for training (i.e., patch-level training data T(₁, ₁₎ corresponding to the first slide for training to pixel-level training data T_{(N, 1 )} corresponding to the Nth slide for training).

In addition, the training module 140 may train the pixel-level classification neural network 170 using N pieces of pixel-level training data (i.e., pixel-level training data T_{(1, 2)} corresponding to the first slide for training to pixel-level training data T_{(N, 12)} corresponding to the Nth slide for training).

Meanwhile, when one round of training for the patch-level classification neural network 160 and the pixel-level classification neural network 170 is completed, the training module 140 may determine whether a predetermined training condition is satisfied, and if satisfied, the training may be terminated (S150).

For example, the training module 140 may determine that the training condition is satisfied when training is repeated a certain number of times. Alternatively, when the trained patch-level classification neural network 160 and/or the pixel-level classification neural network 170 satisfy a predetermined condition, the training module 140 may determine that the training condition is satisfied.

Meanwhile, in an embodiment, the training module 140 may use the patch-level classification neural network 160 that is still in training in the process of generating training data.

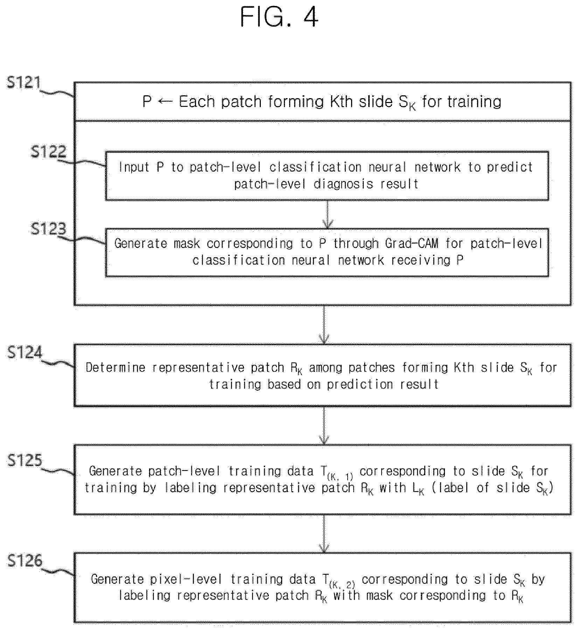

FIG. 4 is a diagram illustrating a specific example of a process (i.e., step S120 of FIG. 3) in which the training module 140 generates training data corresponding to one slide for training (slide S_{K}).

Referring to Figure 4, the training module 140 may input each patch forming the slide S_{K} for training to the patch-level classification neural network 160, and the patch-level classification neural network 160 may predict a patch-level diagnosis result corresponding to each patch (S122).

In addition, the training module 140 may generate a mask image corresponding to each patch through gradient-weighted class activation mapping for the patch-level classification neural network 160 receiving each patch (S123). Gradient-weighted class activation mapping is a technique for generating localization maps that highlight regions that have a significant impact on outcome predictions from images.

For example, the training module 140 may, by masking pixels having a predetermined threshold value or more in the localization map generated by gradient-weighted class activation mapping for the patch-level classification neural network 160 receiving a specific patch, generate a mask image corresponding to the patch.

Meanwhile, the training module 140 may determine the representative patch R_{K} among the patches forming the slides S_{K} for training, based on the diagnosis result for each patch of the patch-level classification neural network 160 performed in step S122 (S124). For example, if the patch-level classification neural network 160 is a neural network that outputs the onset probability of a disease, the training module 140 may determine a patch having the highest onset probability as a representative patch. Alternatively, the training module 140 may determine at least one patch having the onset probability greater than or equal to a predetermined value as representative patches.

Meanwhile, when the representative patch R_{K} is determined in step S124, the training module 140 may label the representative patch R_{K} with the label L_{K} of slide S_{K} for training to generate patch-level training data T_{(K, 1)} corresponding to slide S_{K} (S125).

In addition, the training module 140 may label the representative patch R_{K} with a mask image corresponding to R_{K} to generate pixel-level training data T_{(K, 2 )} corresponding to slide S_{K}.

As described above, according to the training method described with reference to FIGS. 3 and 4, training data for training the patch-level classification neural network 160 and the pixel-level classification neural network are automatically generated from the slide-level training data (i.e., slide images labeled with slide-level diagnosis results), and each neural network is trained therethrough. Accordingly, semi-supervised learning capable of training the patch-level classification neural network 160 and the pixel-level classification neural network using only slide-level training data may be implemented.

In addition, training data is generated based on the prediction result of the patch-level classification neural network 160, and the patch-level classification neural network 160 may be upgraded through gradual training in which the training data generated in this way is used for training the patch-level classification neural network 160 again.

Meanwhile, referring to FIG. 2 again, the annotation module 150 may perform annotation on the biometric image using the pixel-level classification neural network 170 for which training has been completed.

In other words, the annotation module 150 may input a predetermined patch image to be diagnosed to the pixel-level classification neural network 170 on which training is completed, to obtain a classification result for the disease for each pixel forming the patch image to be diagnosed, and may annotate the disease occurred region in the patch image to be diagnosed based on the classification result for each pixel forming the patch image to be diagnosed.

Meanwhile, in the present specification, although an example in which the technical idea of the present disclosure is applied to prostate cancer has been mainly described, an average expert in the technical field of the present disclosure may easily infer that an accurate diagnosis may be made when the technical idea of the present disclosure is applied on not only the specific tissue, but also other diseases that need to be diagnosed in the specific tissue considering the state of the tissue surrounding the tissue.

Meanwhile, according to an embodiment, the diagnosis system 100 may include a processor and a memory storing a program executed by the processor. The processor may include a single-core CPU or a multi-core CPU. The memory may include high-speed random access memory and may also include non-volatile memory such as one or more magnetic disk storage devices, flash memory devices, or other non-volatile solid-state memory devices. Access to memory by processors and other components may be controlled by a memory controller.

Meanwhile, the method according to an embodiment of the present disclosure may be implemented in the form of computer-readable program instructions and stored in a computer-readable recording medium, and a control program and a target program according to an embodiment of the present disclosure may also be stored in a computer-readable recording medium. The computer-readable recording medium includes all types of recording devices in which data that may be read by a computer system is stored.

Program instructions recorded on a recording medium may be those specifically designed and configured for the present disclosure or may be known and available to those skilled in the software.

Examples of computer-readable recording media include magnetic media such as hard disks, floppy disks, and magnetic tapes, optical media such as CD-ROMs and DVDs, magneto-optical media such as floptic disks, and hardware devices specifically configured to store and execute program instructions such as ROM, RAM, flash memory, and the like. In addition, the computer-readable recording medium is distributed in computer systems connected through a network, so that computer-readable codes may be stored and executed in a distributed manner.

Examples of program instructions include high-level language codes that may be executed by a device that electronically processes information using an interpreter, for example, a computer, as well as machine code generated by a compiler.

The hardware device described above may be configured to act as one or more software modules to perform the operations of the present disclosure, and vice versa.

The description of the present invention described above is for illustrative purposes only, and those skilled in the art to which the present disclosure belongs will understand that it may be easily transformed into other specific forms without changing the technical idea or essential features of the present disclosure. Therefore, the embodiments described above should be understood as illustrative in all respects and not limiting. For example, each component described as a single type may be implemented in a distributed manner, and similarly, components described as distributed may also be implemented in a combined form.

The scope of the present disclosure is indicated by the claims described later rather than the detailed description above, and all changes or modifications derived from the meaning and scope of the claims and their equivalent concepts should be construed as being included in the scope of the present disclosure.

### Industrial Applicability

The present disclosure may be used for a method for performing annotations on a pathogenic site of a disease using semi-supervised learning and a diagnosis system for performing the same.

## Claims

1. A method comprising:
generating, by a diagnosis system using a neural network, a patch-level classification neural network configured to receive a patch that is a segmented part of a predetermined slide which is a biometric image and predict a classification result regarding whether or not a predetermined disease exists in the patch, and a pixel-level classification neural network configured to receive the patch and predict a classification result for the disease for each pixel forming the patch;
acquiring, by the diagnosis system using the neural network, a plurality of slide images for training, each of the plurality of slide images for training being labeled with a corresponding slide-level diagnosis result; and
gradually training, by the diagnosis system using the neural network, the patch-level classification neural network and the pixel-level classification neural network using the plurality of slide images for training,
wherein the gradually training of the patch-level classification neural network and the pixel-level classification neural network comprises:
(a) for each of the plurality of slides for training, generating training data corresponding to the slide for training, wherein the training data corresponding to the slide for training comprises patch-level training data for training the patch-level classification neural network and pixel-level training data for training the pixel-level classification neural network;
(b) training the patch-level classification neural network using the patch-level training data corresponding to each of the plurality of slides for training;
(c) training the pixel-level classification neural network using the pixel-level training data corresponding to each of the plurality of slides for training; and
(d) repeatedly performing the operations (a) to (c) at least once.

2. The method of claim 1, wherein the generating of the training data corresponding to the slide for training comprises:
acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes;
determining a representative patch image corresponding to the slide for training among the plurality of patch images corresponding to the slide for training, based on a prediction result for each of the plurality of patch images corresponding to the slide for training; and
labeling the representative patch image corresponding to the slide for training with the slide-level diagnosis result of the slide image for training, thereby generating the patch-level training data corresponding to the slide image for training.

3. The method of claim 1, wherein the generating of the training data corresponding to the slide for training comprises:
acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes;
determining a representative patch image corresponding to the slide for training among the plurality of patch images corresponding to the slide for training, based on a prediction result for each of the plurality of patch images corresponding to the slide for training;
generating a mask corresponding to the representative patch image through gradient-weighted class activation mapping for the classification neural network that output a prediction result for the representative patch image; and
labeling the representative patch image corresponding to the slide for training with the mask corresponding to the representative patch image to generate the pixel-level training data corresponding to the slide for training.

4. The method of claim 1, further comprising:
by inputting a predetermined patch image to be diagnosed into the pixel-level classification neural network for which training has been completed, acquiring a classification result for the disease for each pixel forming the patch image to be diagnosed; and
annotating a pathogenic site of the disease in the patch image to be diagnosed based on the classification result for each pixel forming the patch image to be diagnosed.

5. The method of claim 1, wherein the disease is prostate cancer.

6. A computer program recorded on a non-transitory computer-readable medium for performing the method of any one of claims 1 to 5, which is installed in a data processing device.

7. A diagnosis system using a neural network, comprising:
a processor; and
a memory configured to store a computer program,
wherein the computer program, when executed by the processor, causes the diagnosis system using the neural network to perform the method of any one of claims 1 to 5.

8. A diagnosis system using a neural network, the diagnosis system comprising:
a storage module configured to store a patch-level classification neural network for receiving a patch that is a segmented part of a predetermined slide which is a biometric image and predicting a classification result regarding whether or not a predetermined disease exists in the patch, and a pixel-level classification neural network for receiving the patch and predicting a classification result for the disease for each pixel forming the patch;
an acquisition module configured to acquire a plurality of slide images for training, each of the plurality of slide images for training being labeled with a corresponding slide-level diagnosis result; and
a training module configured to gradually train the patch-level classification neural network and the pixel-level classification neural network using the plurality of slide images for training,
wherein, in order to gradually train the patch-level classification neural network and the pixel-level classification neural network, the training module repeatedly performs a training process two or more times which comprises:
for each of the plurality of slides for training, generating training data corresponding to the slide for training, wherein the training data corresponding to the slide for training comprises patch-level training data for training the patch-level classification neural network and pixel-level training data for training the pixel-level classification neural network;
training the patch-level classification neural network using the patch-level training data corresponding to each of the plurality of slides for training; and
training the pixel-level classification neural network using the pixel-level training data corresponding to each of the plurality of slides for training.

9. The diagnosis system of claim 8, wherein the generating of the training data corresponding to the slide for training comprises:
acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes;
determining a representative patch image corresponding to the slide for training among the plurality of patch images corresponding to the slide for training, based on a prediction result for each of the plurality of patch images corresponding to the slide for training; and
labeling the representative patch image corresponding to the slide for training with the slide-level diagnosis result of the slide image for training, thereby generating the patch-level training data corresponding to the slide image for training.

10. The diagnosis system of claim 8, wherein the generating of the training data corresponding to the slide for training comprises:
acquiring a classification result for each of a plurality of patch images corresponding to the slide for training by inputting each of the plurality of patch images corresponding to the slide for training to the patch-level classification neural network, wherein the plurality of patch images corresponding to the slide for training are a plurality of images obtained by segmenting the slide for training into predetermined sizes;
determining a representative patch image corresponding to the slide for training among the plurality of patch images corresponding to the slide for training, based on a prediction result for each of the plurality of patch images corresponding to the slide for training;
generating a mask corresponding to the representative patch image through gradient-weighted class activation mapping for the classification neural network that output a prediction result for the representative patch image; and
labeling the representative patch image corresponding to the slide for training with the mask corresponding to the representative patch image to generate the pixel-level training data corresponding to the slide for training.

11. The diagnosis system of claim 8, further comprising:
an annotation module configured to:
by inputting a predetermined patch image to be diagnosed into the pixel-level classification neural network for which training has been completed, acquire a classification result for the disease for each pixel forming the patch image to be diagnosed; and
annotate a pathogenic site of the disease in the patch image to be diagnosed based on the classification result for each pixel forming the patch image to be diagnosed.
